# EUROPEAN PATENT SPECIFICATION

(11) **EP 3 639 847 B1**
(45) Date of publication and mention of the grant of the patent: **06.05.2026**
(21) Application number: 19211958.4
(22) Date of filing: 13.03.2013
(51) Int. Cl.: A61K 38/47, A23K 50/20, A23K 20/189, A23K 10/30

(54) **HORSE FEED SUPPLEMENT AND ITS USE**
PFERDEFUTTERZUSATZMITTEL UND DESSEN VERWENDUNG
COMPLÉMENT ALIMENTAIRE POUR CHEVAUX ET SON UTILISATION

(30) Priority: 14.03.2012 GB 201204430
(43) Date of publication of application: 22.04.2020
(62) Divisional of application: 13711456.7
(73) Proprietor: Tharos Limited, Aldridge, Walsall WS9 8LZ (GB)
(72) Inventor: WARING, Rosemary, Birmingham, B45 8NX (GB); HUNTER, John, London, W2 9EE (GB)
(74) Representative: Stratagem IPM Limited

(56) References cited:
- WO-A1-01/49129
- WO-A1-2010/060660
- WO-A2-2007/045450
- DE-A1- 3 028 361
- GB-A- 191 407 742
- HU-B- 203 956
- US-A- 1 019 734
- US-A1- 2009 252 827
- US-B1- 7 005 128
- ANONYMOUS: "Equine Advantage Junior, 50 lb. - Kruse Feed & Supply", 21 April 2009 (2009-04-21), XP055675415, Retrieved from the Internet <URL:https://www.google.com/search?client=firefox-b-ab&biw=1912&bih=1247&tbs=cdr:1,cd_min:2/24/2000,cd_max:3/9/2020&ei=j65nXu2CA4iwkwWWsaiQDw&q=equine+feed+diastatic+malt&oq=equine+feed+diastatic+malt&gs_l=psy-ab.3...13596.14278..14862...1.0..0.126.598.4j2......0....1..gws-wiz.5VecRFD8BdE&ved=0ahUKEwithr> [retrieved on 20200310]
- NERIDA RICHARDS ET AL: "Starch digestion in the equine small intestine - Engormix", 10 February 2006 (2006-02-10), pages 1 - 24, XP055675285, Retrieved from the Internet <URL:https://en.engormix.com/equines/articles/starch-digestion-in-equine-small-intestine-t33469.htm> [retrieved on 20200310]
- DATABASE WPI Week 198217, Derwent World Patents Index; AN 1982-33893E, XP002699996

## Description

The invention relates to an equine feed additive comprising a malt extract for use in the prevention or treatment of an intestinal disease or food digestion associated condition selected from the group consisting of acidosis, colic, diarrhoea and laminitis, wherein the malt extract comprises a plurality of enzymatically active enzymes, wherein the enzymatically active enzymes comprise a plurality of carbohydrases including at least one or more amylases and one or more fructanases, and which is provided in combination with a vegetable material.

Intestinal disease is the biggest cause of mortality and a major cause of morbidity and economic loss in horses. There is a need to be able to identify the health of the intestine of the horse and to improve digestion of food by the horse.

The horse has a digestive strategy that is heavily reliant upon hindgut fermentation of dietary fibre. (C. Janis, Evolution, 1976, 30, 757-774). A consortium of intestinal bacteria in the caecum and colon hydrolyse dietary fibre, releasing soluble carbohydrates which are fermented to short chain fatty acids (acetic, propionic and butyric acid), which provide horses with the majority of their energy requirements. (E.N. Bergman, Physiol. Rev., 1992, 70, 567-590). The energy requirements of horses undertaking heavy work or those performing as athletes far exceed energy arising from hindgut fermentation alone. This "energy gap" is filled by supplementing the diet with readily hydrolysable (digestible) carbohydrate, commonly in the form of grain. However, it is widely recognised that feeding hydrolysable carbohydrate is associated with an increased risk of intestinal disease. (L.L. Clarke et al, Vet. Clin. North Am. Equine Pract. 1990, 6, 433-450) (J.M. Hudson et al, J. Am. Vet. Med. Assoc. 2001, 219, 1419-1425) (S. Gongalves et al, Vet. Res. 2002, 33, 641-652) (M.H. Hillyer et al, Equine Vet. J., 2002, 34, 455-463) (D.C. Archer et al, Vet. J. 2006, 172, 29-39). Acute intestinal disease ("colic") is the single most important cause of mortality in horses and a significant cause of morbidity and economic loss in managed horse populations. (M.K. Tinker et al, Equine Vet J. 1997, 29, 448-453) (J.L. Traub-Dargatz et al, J. A. Vet Med Assoc. 2001, 219, 67-71). Evidence is also emerging of the importance of intestinal disease as a cause of reduced productivity in the 100 million working horses globally. (M.M. Curran et al, Trop Anim Health Prod. 2005, 37, 47-65). Tools allowing the further investigation of relationships between equine health, disease and diet will give rise to welfare, economic and social impact.

There is a growing understanding of the complex interactions between host microbiota and metabolism. (J.K. Nicholson et al, Nat. Rev. Microbiol., 2005, 3, 431-438) (R. Goodacre, J. Nutr., 2007, 137, 259S-266S). Metabolites are highly conserved across species and report directly on the metabolic and physiological status of the subject in health and disease. (E. Holmes et al, Nature, 2008, 453, 396-400) (J.M. Kinross et al, Current Gastroenterology Reports, 2008, 10, 396-403) (J.O. Hunter, Eq. vet. J. 2009, 41, 836-840). The faecal metabolome reports specifically on the metabolic interplay between host, diet and intestinal microbiota and offers the potential to identify biomarkers which can act as proxy for specific bacterial populations. (D.M. Jacobs et al, NMR Biomed., 2008 21, 615-626) (P.J. Turnbaugh et al, Cell, 2008, 134, 708-713).

Volatile organic compounds (VOCs) have been investigated as markers of health or disease. There are many reports of the use of individual VOCs as biomarkers (A. Amann et al, Breath Analysis for Clinical Diagnosis and Therapeutic Monitoring, World Scientific, Singapore, 2005), for example acetaldehyde or formaldehyde as markers of cancer, (D. Smith et al, Rapid Comms in Mass Spectr., 2003, 17, 845-850) (P. Španěl et al, Rapid Comms. in Mass Spectr., 1999, 13, 1354-1359) acetone as a marker of blood glucose in diabetes;( C. Turner, Ex. Rev. of Mol. Diagnost., 2011, 11, 497-503) increased ammonia levels associated with liver or kidney disease. (S. Davies et al, Kidney Intl., 1997, 52, 223-228). In most cases, unique biomarkers are not evident, however by looking at the pattern of biomarkers, the health or disease status of a population may be inferred. In the past this was often done using so called electronic nose (e-nose) technology which is an array of gas sensors, with each sensor being more or less selective for different compounds. Although they do not give information about the VOCs present, they may potentially be used to diagnose disease, for example by testing urine from patients with bladder disease to diagnose cancer. (C. Weber et al, Analyst, 2011, 136, 359-364).

Many animals, including horses and cattle, can have digestive problems when eating carbohydrate-rich food, such as grass in Spring and Autumn, or grain. Attempts have been made to improve the digestion of foodstuffs by the addition of isolated enzymes. Kienzle et al (1994), J. Anim. Physiol. a. Anim. Nutri, 72, 234-241, looked at the activity of amylases in the gastrointestinal tract of the horse. Amylase breaks down starch to sugars. The paper focussed on the activity of amylases produced by the horse. However, the authors of the paper also tried to add bacterially derived amylase to the feed of horses. At very high doses only a small amount (3-4%) of amylase was observed to pass into the jejunum, so did not significantly increase the amount of amylase activity in the gut. Moreover, such bacterial sources of enzymes are relatively expensive and difficult to buy in bulk.

Richards et al (Animal Feed Sci. Technol. (2004) 114, 295-305) also reported the use of exogenous amylase and amyloglucosidase (AMG) to enhance starch digestion in horse. Again bacterially sourced isolated enzymes were studied. AMG had no effect on starch digestion. Amylase initially improved glycaemic responses suggesting improvements in starch suggestion but there was a reduction in response over time.

The inventors realised that digestion might be improved by providing several different enzymes at once, to improve breakdown of plant-based products. Moreover, there was a need to be able to provide the enzymes in a cost effective form to allow the bulk supply of the enzymes as a feedstuff.

Malt is germinated seeds or grain. The seeds are made to germinate by soaking in water and are then usually halted by drying with hot air. Germination causes the seed to produce a variety of enzymes that modify, for example, starches into sugars, through the production of amylases and other carbohydrases, such as fructanases. The germination process also induces other enzymes, such as proteases that break down proteins in the grain. Conventionally malted grains used in the production of beer, whisky, malted vinegar or malt flavourings, are kiln dried at temperatures up to 90°C for ales, 80°C for lagers and lower for malts required to be high in enzyme content. At higher temperatures greater proportions of the enzymes present in the malt are denatured by the heating and drying process.

For example, barley grains are typically grown in a current of cool humidified air and are allowed to germinate ('sprout'). They are then carefully dried ('kilned')and cleaned and the rootlets removed. The sprouted grains are then milled and water is added at the appropriate temperature to make a mash. The various enzymes present are effective at different temperatures, Therefore the mash is usually started at around 50°C when proteases and beta amylases break down the ground malt. The mash temperature is progressively raised to 65°C when the starch gelatinises and permits degradation to sugars by the action of alpha amylases. After mashing and stirring for around 1 hour the liquid is separated from the residual solids ('spent grain'). The liquid ('wort') is then concentrated by vacuum evaporation to give malt extract, typically 80% solids in solution. Malt extract may be made without active enzymes or as an enzyme-rich extract depending on the temperatures used in evaporation. Enzyme-rich extracts have high diastatic power (high DP) and the nature of the enzymes is regulated by the nature of the seed, such as barley variety being malted, the growth conditions during germination and by gentler kilning programmes. The details of the kilning and drying of the malt and of the concentration of the wort determine the colour and flavour of the malt extract and the activity of the enzymes contained in it. The final malt extract is a sugar-rich solution which may, depending on the process used, contain soluble carbohydrate, peptides, amino acids and a range of enzymes. A high DP extract is used for starch degrading activity and enzymes present typically include amylytic activity with α-amylase, β-amylase, debranching enzymes, α-glucosidase, transglucosylases, and phosphorylases. The extract will also typically have significant levels of peptidases and other proteolytic enzymes. Fructosyltransferase enzymes are found in grasses and in cereals including barley (Altenbach et al 2005 FEBS Letters 579 (21) 4647-4653) and as well as being involved in fructan synthesis, have glycoside hydrolase activity to degrade fructans to less complex sugars. Barley leaves contain sucrose-fructan 6-fructosyltransferase which may also be found in malt extract.

### Refs

Briggs DE Bolton CA Brookes PA Stevens R 2009 'Brewing Science and Practice' Woodhead Publishing Abington Hall Cambridge and CRC Press Boca Raton USA
Briggs DE 'Malts and Malting' 1998 Blackie Academic and Professional Publishing London UK

Prior to heating and drying the malt is called "green malt". This is where the grain has germinated but not yet been dried. Typically, for example, seed is immersed or steeped in water two or three times over a period of two or three days to allow the seeds to absorb moisture and start to germinate. The seeds are usually allowed to sprout and occasionally turned for a period of several, typically 3-6 days. At this stage it is called "green malt". After this stage it would normally be dried for conventional use.

Equine Advantage Junior (Kruse Feed & Supply) comprises Saccharomyces Cerevisiae yeast, cane molasses, diastatic malt, corn syrup and dried whey (Internet 21 April 2009).

Richards et al. ('Starch digestion in the equine small intestine: is there a role for supplemental enzymes?', Engomix.com, 10 February 2006) discloses that exogenous enzyme supplementation of equine diets containing a digestible source of starch may be used to overcome endogenous enzyme deficiencies in the equine small intestine, thus removing a major barrier to pre-caecal starch digestion in horses.

WO 2007/045450 (DSM IP Assets B.V.) relates to use of fructanases in feed of hoofed animals, preferably to prevent diseases. In particular, it has been surprisingly found that fructanases such as 2,1-β-D-fructan hydrolase are highly effective as therapeutic and prophylactic agents against laminitis in horses.

US 1 019 734 (F.H. Bennett Biscuit Company) describes a horse food or biscuit and relates particularly to a saccharinated material containing preferably milk in a malted form.

The inventors realised that malted grains allow the production of a ready source of enzymes that might assist in the digestion of foodstuffs in animals.

The invention provides an equine feed additive comprising a malt extract for use in the prevention or treatment of an intestinal disease or food digestion associated condition selected from the group consisting of acidosis, colic, diarrhoea and laminitis, wherein the malt extract comprises a plurality of enzymatically active enzymes, wherein the enzymatically active enzymes comprise a plurality of carbohydrases including at least one or more amylases and one or more fructanases, and which is provided in combination with a vegetable material. That is, the enzymes are still capable of having enzyme activity or a substrate, for example treating starch down into one or more smaller components such as mono- or di-saccharides.

The plurality of carbohydrases are capable of breaking down one or more carbohydrates into smaller components, or proteases. The enzymes may be plant derived, for example derived from germinating seeds.

Amylases catalyse the breakdown of starch into sugars. α-Amylase breaks starch down. It yields maltotriose and maltose from amylose, or maltose, glucose and limit dextrin from amylopectin. β-Amylase breaks starch into maltose. Both α-amylase and β-amylase are found in seeds during germination. Both α- and β-amylase may be present.

Fructanases break down fructans, which are believed to be involved in the induction of laminitis in hoofed animals. Fructanases include2,1-β-D-fructan hydrolases.

Malt is typically green or high diastatic power (high dp) malt. That is from germinated seeds that have not been heated and dried above 40°C, 50°C or 55°C but below 75°C or 70°C to halt germination as this often reduces the activity of enzymes remaining after that heating step. Alternatively malt heated and dried above that temperature may be used, but with lower enzymatic activity.

Typically water is added and heated to at least approximately 40°C or 50°C to form the mash. The temperature may be raised to 55°C, 60°C or 65°C but below 75°C or 70°C and then separated from the grain. The liquid wort may be evaporated by, for example, vacuum evaporation.

A malt extract may be prepared by, for example, crushing the malt and extracting the enzymes with, for example, hot water. The extract may then be evaporated, for example to an 80% sugar solution containing the enzymes (an "amylase rich extract").

Diastatic power is measured in °Lintner (°L) or by Windisch Kolbach Units (°WK). A malt with enough power to self-convert into starch has a diastatic power near 35° Lintner.

A high diastatic malt typically has a value of above 35° Lintner (94° WK), or typically above 45° L, 50° L, 60° L, 70° L or above 80° L.

Potentially any seed may be used to produce the malt. For example, wheat, triticale, sorghum, maize, buck wheat or rice may be used. Barley is typically used as this is regularly used as a source of malt for the brewing industry in many countries. Malt is also tasty to animals such as horses so assists in administering the feed additive.

The addition of malt to animal feed in horses has been shown by the inventors to increase the amount of energy rich fatty acids in the caecum of horses, giving better utilisation of the feed. There is less starch present in the caecum, resulting in the reduced production of D-lactate by bacteria in the gut, resulting in lower acidosis and reduced risk of colic and less diarrhoea.

The ability to increase utilisation of carbohydrates may also be applied to other vegetable eating animals includes ovine, bovine, other equine animals such as zebra, deer, and rabbits.

The animal feed additive may be provided as a separate orally acceptable product, for example in combination with one or more bulking agents, stabilisers, thickeners, additional vitamins, minerals, edible oils (such as linseed oil), salts and/or electrolytes. It may be provided as a liquid, powder or moist product. It may be added to drinking water.

The animal feed additive is provided in combination with vegetable material. This may be a part of the animal's normal feed to prevent, for example colic, or to increase the energy utilisation from the feed. The feed may be, for example, grains such as oats (including bruised, naked or rolled oats), barley or maize; hay such as Timothy hay, or alfalfa hay; or silage. A balancer, such as an oat balancer, may also be provided.

Use of animal feed or drinking water, in combination with the animal feed additive is provided.

The use of the animal feed additive as in an animal feed is also provided.

Amylase rich extracts are typically made by using hot water extraction of germinated barley and evaporation to an 80% sugar solution (D.E. Briggs, The Principles of Mashing In : Malt and brewing, Blackie, 1998).

The amount of food additive provided will vary from animal to animal and food to food. For example, racing horse have been successfully fed 1% body weight of bruised oats, naked oats and oat balancer (ratio 1:0.5:1 w/w. 13MJ/kg dry weight energy density), 1% body weight Timothy hay, 0.15% alfalfa hay plus linseed oil, salt and electrolyte. Daily intake was split between 3 feeds, the evening feed comprising 66% of each horse's daily grain intake. 200 grams of the amylase rich extract was provided. 70% of the daily dose was administered to the larger evening feed.

The inventors studied the effect of the feed additive on Volatile Organic Compounds (VOCs) in the faeces of horses. They found that the VOCs found prior to adding the feed additive compared to that after the feed additive changed. The inventors found a decrease in organic acids, alcohols and ketones associated with bacterial digestion of carbohydrate in the hindgut. This suggested that the feed additive enhanced pre-caecal digestion. It also suggested that VOCs are a way of assessing the health of the gut.

A method of monitoring the health of the gut of an equine animal is disclosed, comprising measuring the concentration of one or more volatile organic compounds from the faeces of an animal selected from acetic acid, butanoic acid, propanoic acid, 2-methyl propanoic acid and 3-methyl butanoic acid.

One, two, three, four or all five compounds may be measured. One or more of 4-methyl phenol, acetone and/or isopropyl alcohol may be measured.

The faeces are typically fresh or fresh faeces frozen substantially afterwards.

The amount may be compared to an amount measured prior to administration of the additive. It may also be used to determine whether an animal should be treated with, for example, the additive.

A computer implemented method for determining in a sample of faeces the concentration of one or more VOCs by the method and comparing to a predetermined normal value is disclosed. This gives an indication of the relative health of the animal.

Apparatus comprising a computer processor and a memory configured to carry out the method is disclosed.

The invention will now be described by way of example only with reference to the following figures:
**Figure 1** : A schematic diagram of the SIFT-MS instrument indicating the main components. Headspace samples of faeces in a Nalophan bag may be analysed as illustrated, by attaching the bag to the inlet capillary of the instrument.
**Figure 2** **:** Univariate analysis of eight most abundant volatile organic compounds by GC-MS in faeces of Thoroughbred racehorses. Group identification : 1 = horses in race training pre-supplementation (n=8); 2 = horses in race training after six weeks supplementation (n=6). A=acetic acid; B=butanoic acid; C-propranoic acid; D-propanoic acid 2 methyl; E=butanoic acid 3 methyl; F=phenol4 methyl; G=acetone; H=isoproyl alcohol.
**Figure 3** : Scores plots for multivariate analysis of faecal SIFT-MS data from racehorses pre-(labelled "na") and post-supplementation (labelled "nb"); A) PCA model, B) OPLS-DA model. (Post-supplementation plots to the left of the centre y axis.)
**Figure 4** : S-plot (A) and loadings plot with 95% confidence intervals (B) for the OPLS-DA model describing faecal VOCs in horse faces before and after supplementation with enzyme-rich malt.

### Material and Methods

### Study population and sampling

The study population comprised 8 castrated male (gelding), Tb racehorses between the ages of 4 and 6 years old. Each horse was stabled at the same premises, under the management of the same trainer and in active race training. All horses were free from concurrent medical treatment throughout the study. All horses were fed a standard diet comprising 1% bodyweight fed as a mix of bruised oats, naked oats and oat balancer (ratio of 1:0.5:1 respectively, 13MJ/kg dry weight energy density), 1% bodyweight Timothy hay, 0.15% bodyweight alfalfa hay plus linseed oil, salt and electrolyte supplementation. Daily intake was divided between three feeds, the evening feed comprising 66% of each horse's daily concentrate (grain) intake. All horses in this population were sampled once before and once after six weeks supplementation of their diet with amylase-rich malt extract (Muntons, Stowmarket, UK). Briefly, this is prepared by hot water extraction of maltose from barley, evaporation to an 80% sugar solution and conversion of starch to simple sugars by amylolytic enzymes which are present in excess (D.E. Briggs, The Principles of Mashing In: Malt and Brewing, Blackie, 1998). The rationale for this supplement is that small intestinal amylase production is known to be low in the horse compared to other species resulting in a "bottleneck" for carbohydrate digestion. (E. Kienzles et al, J. An. Physiol. An. Nutr., 1994, 72, 234-241). Exogenous dietary amylase from bacteria previously has been shown to enhance equine carbohydrate digestion (N. Richards et al, An. Feed Sci. Technol. 2004, 114, 295-305). Two hundred grams of amylase-rich malt extract was added to morning and evening feeds, 70% of the daily dose being administered to the larger evening feed. All sampling took place in the morning, immediately following exercise, approximately 3 hours after the horses received a small meal. Horses were excluded from the study if they were receiving any concurrent medication or if they were unavailable for sampling. Sampling and metadata collection were carried out under University of Liverpool ethics approval RETH000363, with the informed consent of the trainer. Freshly voided faeces were collected into polyethylene sample containers and immediately frozen in liquid nitrogen prior to transfer into a freezer at -80 °C.

### Headspace preparation

Frozen faecal samples were defrosted at room temperature. Five grams of each sample was removed from the bulk sample and placed inside a Nalophan sampling bag, made up of 65 mm diameter Nalophan NA tubing 25 µm thick. The sample bags were 40 cm long. One end of the bag was fitted with a Swagelok connector and the other end of the bag was sealed and filled with hydrocarbon free air to generate the headspace of VOCs. The bags were then placed in an incubator at 40°C for 1 hour to allow the volatile organic compounds (VOCs) to equilibrate between headspace and solid sample.

After 1 hour, the headspace was analysed by connecting the Swagelok fitting to the capillary inlet of a selected ion flow tube mass spectrometry (SIFT-MS). 200ml of headspace was then pumped across a thermal desorption (TD) tube for thermal desorption - gas chromatography - mass spectrometry (TD-GC-MS) as described below.

SIFT-MS (see below) is a real time trace gas analyser which can accurately quantify the concentration of trace gases present. The model used in this study (Mk 2) can typically detect down to a level 10 parts-per-billion (ppb). GC-MS, on the other hand, is much slower but when combined with TD offers the ability to detect VOCs at the parts per trillion (ppt) level. As it is not a direct method of analysis, it is also less quantitative than the direct SIFT-MS method.

### SIFT-MS

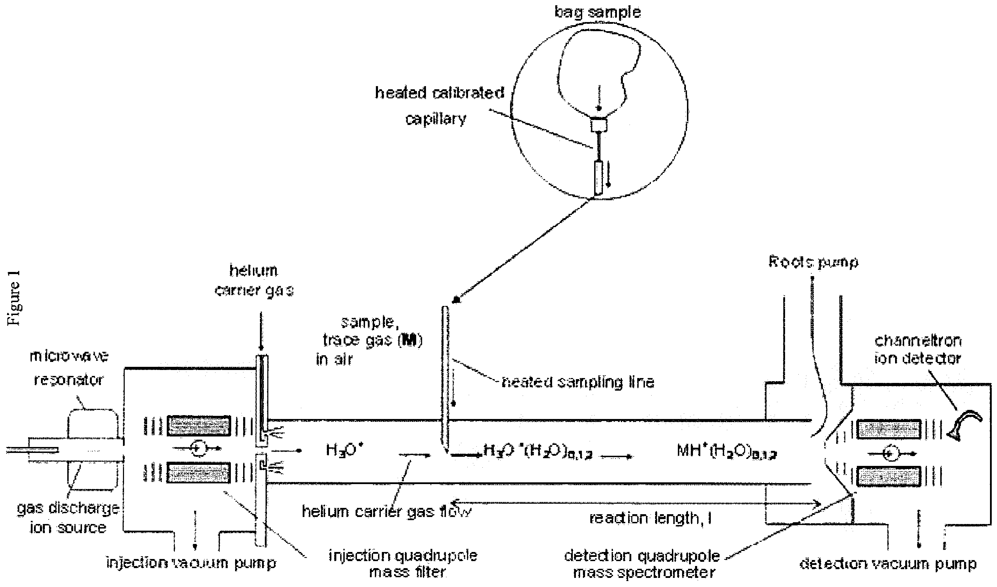

SIFT-MS has been described in detail previously (A. Amann et al, Breath Analysis for Clinical Diagnosis and Therapeutic Monitoring, World Scientific, Singapore, 2005) (D. Smith et al, Mass Spectrom. Rev., 2005, 24, 661-700) so only a brief summary is given here. Analysis requires the generation of precursor ions which are produced in a microwave discharge and are selected by the first of two quadrupole mass filters before being injected into a fast flowing helium carrier gas (Fig. 1). These ions then react with the VOCs in the sample which is drawn into the flow tube via a heated capillary inlet. The available precursor ion species are H₃O⁺, NO⁺ and O₂⁺. The precursor and product ions in the carrier gas are sampled by a downstream orifice and pass into a differentially pumped second quadrupole mass spectrometer and ion counting system for analysis.

Full spectra of the count rates at each m/z value in the range m/z 10 to m/z 140 were recorded for all the samples using each precursor ion. The identities and concentrations of various components were determined using the on-line database containing reaction rate coefficients, developed from numerous detailed selected ion flow tube (SIFT) studies of various classes of compounds (alcohols, aldehydes, ketones, hydrocarbons, etc) with the three precursor ions. (D. Smith et al, Mass Spectrom. Rev., 2005, 24, 661-700) (D. Smith et al, Rapid Comms. in Mass Spectr., 1996, 10, 1183) (P. Španěl et al, Int. Rev. Phys. Chem., 1996, 15, 231-271).

SIFT-MS data were normalised to the precursor ions for each set of spectra in order to account for the variability in ion count. These data were then exported to spread sheet software for further processing and removal of zero values. The data were then imported into SIMCA-P+ as described below.

### TD-GC-MS

The Nalophan bags were connected to a TD tube for subsequent analysis by GC-MS to pre-concentrate the headspace via an automated pump using 200 ml of faecal headspace. Standard stainless-steel TD sorbent cartridges were used, containing dual packing comprising 50% Tenax TA and 50% Carbotrap (Markes International Limited, Llantrisant, UK). Cartridges were conditioned before use by purging with helium carrier gas for 2 min at room temperature followed by 1 hour at 320 °C.

Captured volatiles were analysed using an AutoSystem XL gas chromatograph equipped with an ATD 400 thermal desorption system and TurboMass mass spectrometer (Perkin Elmer, Wellesley, MA). CP grade helium (BOC gases, Guildford, UK) was used as the carrier gas throughout, after passing though a combined trap for the removal of hydrocarbons, oxygen and water vapour. Cartridges were desorbed by purging for 2 min at ambient temperature then for 5 min at 300 °C. Volatiles purged from the cartridge were captured on a cold trap which was initially maintained at -30 °C. Once desorption of the cartridge was complete, the trap was heated to 320 °C using the fastest available heating rate and maintained at that temperature for 5 minutes whilst the effluent was transferred to the gas chromatograph via a heated (180 °C) transfer line coupled directly to the chromatographic column.

A Zebron ZB624 chromatographic column (dimensions 30m × 0.4mm × 0.25mm ID) was used (Phenomenex, Torrance, CA). The gas chromatograph oven was maintained at 50 °C for 4 min following injection and was then raised at 10 °C.min⁻¹ to 220 °C for 9 min. Separated products were transferred by heated line to the mass spectrometer and ionised by electron bombardment. The spectrometer was set to carry out a full scan from mass/charge ratios (m/z) 33 to 350 using a scan time of 0.3s with a 0.1s scan delay. The resulting mass spectra were combined to form a total ion chromatogram (TIC) by the GCMS integral software (TuboMass ver 4.1) and resolved compounds were identified usingAMDIS software and the NIST mass spectral database.

**Table 1. Prevalence of VOC's in faecal headspace (% horses positive) detected by GC-MS, for each group of horses; NA = pre-supplementation racehorses, NB = post-supplementation racehorses.**

| **Headspace VOC** | **NA(n=8)** | **NB(n=6)** |
|---|---|---|
| 1,3-Dioxolane, 2-methyl- | 100 | 100 |
| 2-Butanol | 100 | 67 |
| 2-Butanone | 100 | 100 |
| Acetic acid | 100 | 100 |
| Acetone | 100 | 100 |
| Butanoic acid | 100 | 83 |
| Phenol, 4-methyl- | 100 | 100 |
| Propanoic acid | 100 | 83 |
| Tetradecane | 100 | 50 |
| 2,3-Butanedione | 88 | 100 |
| 2-Propenoic acid, 2-methyl-, methyl ester | 88 | 67 |
| Butanoic acid, 2-methyl- | 88 | 67 |
| Butanoic acid, 3-methyl- | 88 | 67 |
| Isopropyl Alcohol | 88 | 100 |
| Pentanoic acid | 88 | 33 |
| Propanoic acid, 2-methyl- | 88 | 67 |
| R(-)3,7-Dimethyl-1,6-octadiene | 88 | 100 |
| Toluene | 88 | 100 |
| Butanal, 2-methyl- | 75 | 100 |
| Butanal, 3-methyl- | 75 | 100 |
| Disulfide, dimethyl | 75 | 83 |
| Pentane | 75 | 100 |
| 1-Butanol | 63 | 67 |
| 2,4-Dimethyl-1-heptene | 63 | 50 |
| Acetaldehyde | 63 | 83 |
| Dimethyl trisulfide | 50 | 50 |
| Hexanoic acid | 50 | 0 |
| Propanal, 2-methyl- | 50 | 50 |
| 4 methyl heptane | 50 | 0 |
| 1,6-Octadiene, 3,7-dimethyl-, (S)- | 38 | 0 |
| 1-Propanol | 38 | 100 |
| Ethyl alcohol | 38 | 100 |
| Hexanal | 38 | 67 |
| Nonanal | 38 | 33 |
| Undecane | 38 | 0 |
| 1-Pentanol | 25 | 67 |
| 1-Propanol, 2-methyl- | 25 | 33 |
| 2-Butanone, 3-hydroxy- | 25 | 67 |
| Benzaldehyde | 25 | 67 |
| Phenol | 25 | 50 |
| Nonane | 25 | 0 |
| Dodecane | 17 | 0 |
| Hexane | 17 | 13 |
| Hexane, 2,4-dimethyl- | 17 | 0 |
| Tridecane | 17 | 0 |
| 2-Pentanone | 13 | 0 |
| 2-Propenoic acid, 2-methyl-, 3-hydroxypropyl ester | 13 | 0 |
| Acetic acid, hydrazide | 13 | 33 |
| Dimethyl sulfone | 13 | 0 |
| Furan, tetrahydro- | 13 | 17 |
| Methacrolein | 13 | 33 |
| Octanal | 13 | 0 |
| Pentanal | 13 | 33 |
| Propanal | 13 | 67 |
| Styrene | 13 | 0 |
| 2 methyl nonane | 13 | 0 |
| 2 methyl pentane | 13 | 17 |
| 2,3 dimethyl hexane | 13 | 0 |
| 1,7-Octadiene, 2,7-dimethyl- | 0 | 17 |
| 1-Butanol, 2-methyl-, (S)- | 0 | 0 |
| 1-Butanol, 3-methyl- | 0 | 33 |
| 1-Pentene, 2-methyl- | 0 | 50 |
| 2-Butene | 0 | 17 |
| 2-Propanol, 1,1,1,3,3,3-hexafluoro- | 0 | 0 |
| 2-Propanol, 2-methyl- | 0 | 67 |
| 3-Carene | 0 | 0 |
| á-Pinene | 0 | 17 |
| Benzene | 0 | 17 |
| Butanal | 0 | 0 |
| Decanal | 0 | 17 |
| Dimethyl sulfide | 0 | 50 |
| D-Limonene | 0 | 0 |
| Heptane, 4-methyl- | 0 | 50 |
| Propane, 2-ethoxy-2-methyl- | 0 | 17 |
| 3 hexanone | 0 | 17 |
| 3 methyl hexane | 0 | 33 |
| Decane | 0 | 0 |
| Heptane, 4-methyl | 0 | 50 |
| 1,3-Dioxolan-2-one | 0 | 17 |

### Univariate data analysis

Data were classified as pre-supplementation (NA) and post-supplementation (NB). GC-MS analytes for all subjects were ordered by abundance. Descriptive statistics were generated for each group to evaluate the assumption of a Normal distribution of data and outliers were identified as any observations exceeding two standard deviations from the median. Box and whisker plots were generated for the eight most abundant compounds, grouped by classification, and the significance of inter-group variation assessed by Wilcoxon rank-sum test, a P-value of less than 0.05 indicating a significant difference between group means.

### Multivariate analysis

Normalised SIFT-MS data were converted into spreadsheet format and imported into SIMCA-P+ (version 12, Umetrics, Umea, Sweden) for multivariate analysis. Pareto scaling of data was applied. A principal component analysis (PCA) model was generated for the racehorse population pre- and post-diet supplementation. The first two components were fitted and scores plots generated. Significant outliers were identified using Hotelling's T² range, observations outside the 95% critical limit were excluded from further analysis. Model fit was described by *R²*, the proportion of variance in the data explained by the model. Predictive ability of the model was explained by *Q²*, the proportion of variation predicted by the model in a seven group cross-validation.

Associations between variables and class/group (pre- vs. post diet supplementation) were explored with an OPLS (orthogonal partial least squares) model in order to maximise separation of observations based on class and to measure non-correlated (orthogonal) variation. As for PCA models, Hotellings T² range was used on scores plots to evaluate outliers, the quality of model fit by *R²*, and model predictive ability by *Q²*. An S-plot of loadings was used to evaluate the magnitude (x-axis) and reliability (y-axis) of each variable (m/z value) in predicting class assignment. Jack-knifed 95% confidence intervals were added to a loadings bar chart to evaluate the significance of association between each variable and class assignment.

### Results

### Univariable analysis of faecal GC-MS data

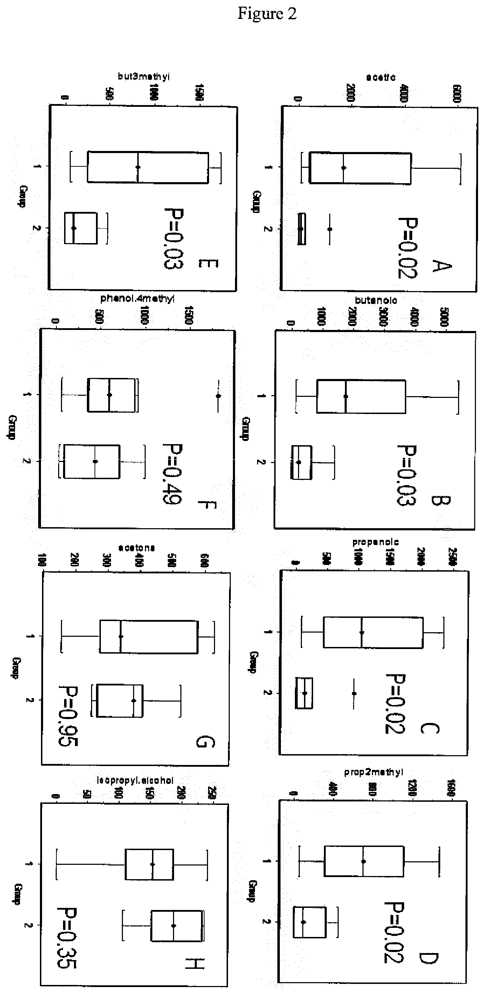

Table 1 lists the identity of metabolites detected by GC-MS from the faeces of our population pre- and post-supplementation. Figure 2 illustrates the distribution of abundance measurements for the eight most abundant compounds detected by GC-MS. For acetic, butanoic and propanoic acid, propanoic acid 2 methyl and butanoic acid 3 methyl, median abundance is significantly reduced in faeces post-supplementation compared to pre-supplementation (P<0.05).

### Multivariable analysis of faecal SIFT-MS data

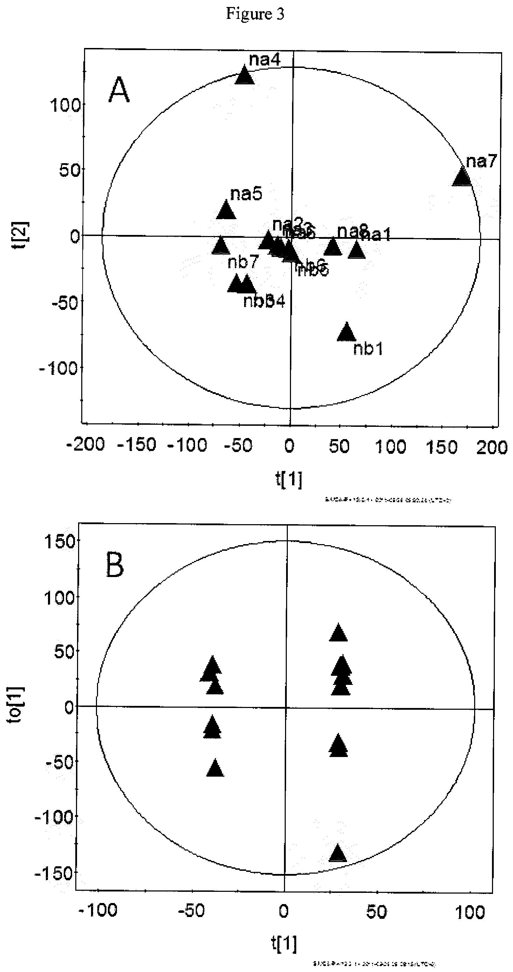

A PCA model for faecal SIFT-MS data (with an H₃O⁺ carrier ion) demonstrated poor model fit and very poor predictive ability, with an *R²* value of 0.37 and *Q²* value of -0.07. A scores plot of the first two components of this model is shown in Fig 3A, with the 95% confidence range for Hotellings *T²* plotted as an ellipse. Although two observations lie close to this limit, they are not outside of the range and therefore were not excluded from further analysis. The same data were modelled using an OPLS model, a scores plot is illustrated in figure 3B. The model was a good fit with good predictive ability (*R²* = 0.85 and *Q²* = 0.74); a scores plot of the first two components demonstrates good separation based on class (Fig. 3B).

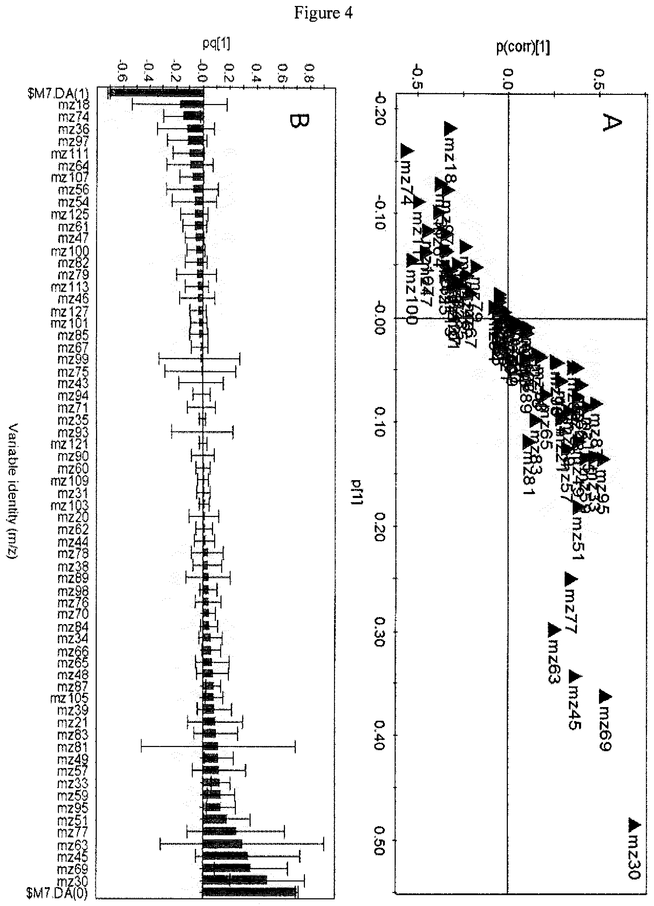

An S-plot of loadings for the OPLS model (Fig 4A) identifies some compounds to be strongly associated with class, the significance of the association for each is explored with 95% confidence intervals in figure 4B. The following ions are significantly associated with class i.e. differentiate between pre- and post-supplementation faeces: m/z 33, m/z 59, m/z 69, m/z74 and m/z 95. Most likely identification of each compound is: m/z 33: methanol; m/z 59: acetone; m/z 69 methanol (with 2 hydrates), a consequence of samples being moist (N. Richards, M. Choct, G.N. Hinch and J.B. Rowe, An. Feed Sci. Technol. 2004, 114, 295-305) m/z 95: acetone (with 2 hydrates). Thus using the H₃O⁺ carrier ion for SIFT-MS analysis, acetone and methanol appear to be significantly associated with class.

### Discussion

This study is the first to report the volatile metabolite profile of equine faeces measured by SIFT-MS and GC-MS. Unsurprisingly, the catalogue of volatile organic compounds detected in equine faecal headspace is dominated by acids, alcohols and ketones, most likely arising from bacterial digestion of carbohydrate including dietary fibre (table 1). A major difference between the faecal VOC metabolome of the horse and the human is the lower frequency of detection of sulphide and disulphide compounds. Dimethyl sulphide was infrequently detected in horse faecal headspace but was present in most human samples (C.E. Garner, S. Smith, B. de L. Costello, P. White, R. Spencer, C.S.J. Probert, and N.M. Ratciffe FASEB J, 2007, 21, 1675-1688). Dimethyl disulphide was ubiquitous in human faecal headspace, it was detected in many samples in the present study, but not all. These observations suggest differences in sulphur metabolism between human and equine intestinal tracts.

In contrast to human faecal headspace, a very limited range of aromatic compounds was identified. Toluene and 4-methyl-phenol are the two most frequently detected aromatic compounds in horse faecal headspace (found in nearly 97% of samples) with tetrahydro-furan, styrene and benzene being present in a small number of samples. In human faecal headspace 56 different aromatics have been detected with many being present in 50% of their samples or more. (C.E. Garner et al, FASEB J, 2007, 21, 1675 - 1688). The same study also reported detecting many alkenes including some with high frequency e.g. limonene was found in nearly all samples from normal humans. Alkenes (2-butene, 3-carene, a-pinene and limonene) were only occasionally found in our equine samples.

Also of note is the marked difference in VOC diversity observed in our equine samples compared to human faecal samples. (C.E. Garner et al, FASEB J, 2007, 21, 1675 - 1688). We detected 81 different VOC's in 20 different samples, Garner *et al.* reported 297 VOC's in 151 samples. A further difference is that in the human study 78 VOC's were present in 50% or more of the samples from non-diseased individuals; in our equine study only 28 VOC's fulfil this criterion. Whilst differences exist in sampling method, sample size and method of VOC analysis, this observation does suggest that a more limited repertoire of VOC's may be present in horse faeces. This may be related to the evolutionary adaptation of intestinal microbiota to a herbivorous diet, to the limited diet (i.e. oats, dry forage and oil) of the horses in our study, and to the similarity in diet between all study horses.

In our Tb racehorse study population faecal VOC measurement was a sensitive discriminator between faeces from pre- and post-supplementation horses. Univariable analysis of GC-MS data indicates a significant decrease in many of the products of carbohydrate digestion following supplementation with amylase-rich malt extract. From this study we are unable to establish whether this is the result of increased absorption or decreased production. However, we believe that in this population which is fed a carbohydrate-rich diet, the observed effect is due to enhanced pre-caecal digestion of starch by exogenous amylase resulting in less dietary carbohydrate reaching the hindgut for microbial fermentation. (E. Kienzles et al, J. An. Physiol. An. Nutr., 1994, 72, 234-241) (R.M. Hoffman R. Bras. Zootec., 2009, 38, 270-276). The potential importance of pre-caecal carbohydrate digestion in human and animal intestinal health has been highlighted by one of the authors (J.O. Hunter Lancet 1991, 338, 495-6). Further studies will be necessary to test this hypothesis.

An OPLS discriminant analysis demonstrated clear separation of pre- and post-supplementation samples based on SIFT-MS metabolic profile (Fig 3B). This finding indicates the sensitivity of faecal SIFT-MS for the evaluation of equine diet, dietary supplements, and the evaluation of intestinal health. Combined with the ease of sample collection and processing, this experimental model has demonstrated considerable utility which, with further refinement, may lead to novel strategies for monitoring equine intestinal health.

Our observations of significant differences in faecal methanol and acetone measurements following supplementation suggest that these VOC's are worthy of further investigation as potential biomarkers for intestinal health in horses. SIFT-MS is especially useful for looking at low molecular mass compounds; some of which could not be detected using the TD-GC-MS method used. SIFT-MS is better at obtaining quantitative data as the headspace is analysed directly. However, the greater sensitivity and identification ability of TD-GC-MS enables a much more effective qualitative investigation into the compounds present. The combination of the two techniques is an especially powerful strategy for investigating the faecal metabolome.

### Conclusions

We conclude that the TD-GC-MS and SIFT-MS metabolic profile of Tb racehorse faeces is dominated by acids, alcohols and ketones, most likely derived from hindgut microbial digestion of carbohydrate. Sulphur-containing VOC's were detected less frequently and a limited number of aromatic compounds were present compared to human faeces.

Multivariate analysis indicates that acetone was significantly increased in quantity and methanol significantly decreased in faeces after dietary supplementation with amylase-rich malt extract. These two compounds are worthy of further investigation as biomarkers for equine intestinal health. In evaluating acetone data, SIFT-MS gave more accurate results than TD-GC-MS as acetone was analysed directly; the TD sorbents used are better suited to higher molecular weight compounds. Our study demonstrates the utility and sensitivity of an analytical model for investigating the metabolic consequences of dietary supplementation in horses.

## Claims

1. An equine feed additive comprising a malt extract for use in the prevention or treatment of an intestinal disease or food digestion associated condition selected from the group consisting of acidosis, colic, diarrhoea and laminitis, wherein the malt extract comprises a plurality of enzymatically active enzymes, wherein the enzymatically active enzymes comprise a plurality of carbohydrases including at least one or more amylases and one or more fructanases, and which is provided in combination with a vegetable material.

2. An equine feed additive comprising a malt extract for use in the prevention or treatment of an intestinal disease or food digestion associated condition selected from the group consisting of acidosis, colic, diarrhoea and laminitis according to claim 1, wherein the vegetable material is selected from one or more of grains including oats, barley or maize; hay including Timothy hay or Alfalfa hay; and silage

3. An equine feed additive comprising a malt extract for use in the prevention or treatment of an intestinal disease or food digestion associated condition selected from the group consisting of acidosis, colic, diarrhoea and laminitis according to claim 1 or claim 2, wherein the malt extract has a diastatic power of above 35° Lintner (L) (94°WK), 45°L, 50°L, 60°L, 70°L or 80°L.

## Patentansprüche

1. Pferdefutterzusatzstoff, umfassend einen Malzextrakt zur Verwendung bei der Vorbeugung oder Behandlung einer Darmerkrankung oder eines mit Nahrungsverdauung assoziierten Zustands ausgewählt aus der Gruppe bestehend aus Azidose, Kolik, Durchfall und Laminitis, wobei der Malzextrakt eine Vielzahl von enzymatisch aktiven Enzymen umfasst, wobei die enzymatisch aktiven Enzyme eine Vielzahl von Kohlenhydrasen umfassen, beinhaltend zumindest eine oder mehrere Amylasen und eine oder mehrere Fruktanasen, und der in Kombination mit einem pflanzlichen Material bereitgestellt ist.

2. Pferdefutterzusatzstoff, umfassend einen Malzextrakt zur Verwendung bei der Vorbeugung oder Behandlung einer Darmerkrankung oder eines mit Nahrungsverdauung assoziierten Zustands ausgewählt aus der Gruppe bestehend aus Azidose, Kolik, Durchfall und Laminitis nach Anspruch 1, wobei das pflanzliche Material ausgewählt ist aus einem oder mehreren von Körnern, beinhaltend Hafer, Gerste oder Mais; Heu, beinhaltend Timothy-Heu oder Alfalfa-Heu; und Silage.

3. Pferdefutterzusatzstoff, umfassend einen Malzextrakt zur Verwendung bei der Vorbeugung oder Behandlung einer Darmerkrankung oder eines mit Nahrungsverdauung assoziierten Zustands ausgewählt aus der Gruppe bestehend aus Azidose, Kolik, Durchfall und Laminitis nach Anspruch 1 oder Anspruch 2, wobei der Malzextrakt eine diastatische Wirkung von über 35° Lintner (L) (94° WK), 45° L, 50° L, 60° L, 70° L oder 80° L aufweist.

## Revendications

1. Additif alimentaire équin comprenant un extrait de malt destiné à être utilisé dans la prévention ou le traitement d'une maladie intestinale ou d'un problème associé à la digestion alimentaire choisi dans le groupe constitué par l'acidose, la colique, la diarrhée et la laminite, dans lequel l'extrait de malt comprend une pluralité d'enzymes actives sur le plan enzymatique, dans lequel les enzymes actives sur le plan enzymatique comprennent une pluralité de carbohydrases comprenant au moins une ou plusieurs amylases et une ou plusieurs fructanases, et qui est fourni en combinaison avec une matière végétale.

2. Additif alimentaire équin comprenant un extrait de malt destiné à être utilisé dans la prévention ou le traitement d'une maladie intestinale ou d'un problème associé à la digestion alimentaire, choisi dans le groupe constitué par l'acidose, la colique, la diarrhée et la laminite selon la revendication 1, dans lequel la matière végétale est choisie parmi un ou plusieurs éléments parmi des grains, y compris l'avoine, l'orge ou le maïs ; du foin, y compris le foin de Timothy ou le foin de luzerne ; et un produit d'ensilage.

3. Additif alimentaire équin comprenant un extrait de malt destiné à être utilisé dans la prévention ou le traitement d'une maladie intestinale ou d'un problème associé à la digestion alimentaire choisi dans le groupe constitué par l'acidose, la colique, la diarrhée et la laminite selon la revendication 1 ou la revendication 2, dans lequel l'extrait de malt présente un pouvoir diastatique supérieur à 35° Lintner (L) (94 °WK), 45 °L, 50 °L, 60 °L, 70 °L ou 80 °L.
